# EUROPEAN PATENT SPECIFICATION

(11) **EP 1 242 073 B1**
(45) Date of publication and mention of the grant of the patent: **22.09.2004**
(21) Application number: 00973658.8
(22) Date of filing: 19.10.2000
(51) Int. Cl.: A61K 31/4015

(54) **COMPOSITIONS FOR PREVENTION AND TREATMENT OF COLD AND INFLUENZA-LIKE SYMPTOMS AND THEIR METHODS OF USE**
ZUSAMMENSETZUNGEN ZUR VORBEUGUNG UND BEHANDLUNG DER ERKÄLTUNG UND GRIPPE-ARTIGER SYMPTOME UND IHRE VERWENDUNG
COMPOSITIONS DESTINEES A LA PREVENTION ET AU TRAITEMENT DU RHUME ET DES SYMPTOMES DE TYPE GRIPPAL ET PROCEDES D'UTILISATION DE CES DERNIERES

(30) Priority: 19.10.1999 US 421131
(43) Date of publication of application: 25.09.2002
(73) Proprietor: THE PROCTER & GAMBLE COMPANY, Cincinnati, Ohio 45202 (US)
(72) Inventor: RENNIE, Paul John, Godalming, Surrey GU7 2HA (GB); KING, Simon, Phillip, Weybridge KT13 8TJ (GB); BIEDERMANN, Kimberly, Ann, Cincinnati, OH 45241 (US); MORGAN, Jeffrey, Michael, Springboro, OH 45066 (US)
(74) Representative: Clemo, Nicholas Graham
(86) International application number: PCT/US2000/028856
(87) International publication number: WO 2001/028556

(56) References cited:
- EP-A- 0 371 421
- EP-A- 0 408 330
- EP-A- 0 856 316
- DE-A- 3 431 727
- US-A- 4 444 885
- US-A- 4 767 788
- US-A- 5 508 282

## Description

### FIELD OF USE

The present invention is for compositions and their methods for prevention and treatment of cold and influenza-like symptoms due to respiratory tract viral infections. These compounds and their method of application are effective in both preventing the onset of the symptoms of colds and influenza or significantly mitigating them if already afflicted with such symptoms.

### BACKGROUND SUMMARY OF THE ART

It is known that many different viruses and viral strains bring on symptoms associated with respiratory viral infections. The common cold is a complex syndrome caused by over 200 antigenically different viruses found in five virus families. These families include rhinovirus, myxovirus, paramyxovirus, respiratory syncytial virus, adenovirus and coronavirus. The most important group is rhinovirus, Gwaltney J.M., Common Cold, pp 489-493, Mandell G.L., Douglas, R.G. Jr., Bennett, J.E., Principles and Practice of Infectious Diseases, 3^{rd} ed., Churchill Livingstone, New York, 1990. Pinpointing the specific cause of the illness is difficult and not practical since there are also a number of predisposing factors whose contribution to the manifestation of symptoms is not fully understood. Such include, but, are not limited to physical fatigue, psychological stress, and overall physical healthiness.

Regardless of the virus and associated factors leading to the onset of cold and influenza symptoms, a number of remedies to alleviate the symptoms of the common cold have been suggested. The cough/cold products that are currency marketed typically contain one or more of the following actives: nasal decongestants such as pseudoephedrine, oxymetazoline, antihistamines such as doxylamine, antitussives such as dextromethorphan, expectorants such as guaifenesin and anti-pyretics such as acetaminophen. In an attempt to improve existing cold remedies, experts in the field have suggested several alternative pharmacotherapies and subsequently conducted cold trials to test their efficacy. Examples of these therapies include: the use of interferon-α₂, Douglas et al., Prophylactic Efficacy of Intranasal Alpha₂- Interferon Against Rhinovirus Infection in the Family Setting, The New England Journal of Medicine, 314, pp. 65-70, 1986; bradykinin antagonist, Higgins et al., A Study of the Efficacy of the Brandykinin Antagonist, NPC567, in Rhinovirus Infections in Human Volunteers, Antiviral Research vol. 14, pp. 339-344, 1990; glucocorticoid, Farr et al., A Randomized Controlled Trial of Glucocorticoid Prophylaxis Against Experimental Rhinovirus Infection, Journal of Infectious Diseases, vol. 162, pp. 1173-1177, 1990; nedocromil, Barrow et al., The Effect of Intranasal Nedocromil Sodium on Viral Upper Respiratory Tract Infections in Human Volunteers, Clinical and Experimental Allergy, vol. 20, pp. 45-51, 1990; a combination of interferon-α₂, ipratropium and naproxen, Gwaltney, Combined Antiviral and Antimediator Treatment of Rhinovirus Colds, The Journal of Infectious Diseases vol. 166, pp. 776-782, 1992; zinc salts, Potter et al., DIAS Rounds, Zinc Lozenges for Treatment of Common Colds, The Annals of Pharmacotherapy, vol. 27, pp. 589-592,1993.

A number of patents have also been issued disclosing compositions for prevention and treatment of the common cold and their methods of use. A sample of such patents include:US Patents 5,240,694; 5,422,097; and 5,492,689; all to Gwaltney, disclosing treatment using combinations of anti-viral and anti-inflammatory compounds; US Patents Re 33,465 and 5,409,905; both to Eby disclosing treatment using zinc salts; US Pat. 5,626,831; to Van Moerkerken disclosing treatments using orally administered aminocarboxylic acid compounds; U.S. Patents 4,619,934 and 4,552,899, both to Sunshine, disclosing treatment of cough and colds using compositions comprising non-steroidal anti-inflammatory drugs such as NSAIDS with antihistaminically effective materials such as chlorpheniramine; US 5,508,282 disclosing pH 5.5-6.5 ascorbic acid and caffeine compositions; DE 3,431,727 teaching zinc gluconate nasal sprays; EP 408,330, relating to silver and zinc salts of fusidic acid; and EP 856,316 which discloses near neutral nasal drops comprising a pyridone carboxylic acid.

Treatment for influenza includes vaccination and use of specific antiviral drugs. These have been reviewed by A. Elliot and J. Ellis, 2000, Pharmaceutical Journal, 265, 446-451. Amantidine and Rimantidine have been used for treating influenza infections. They target the M2 protein of influenza virus and interfere with release of viral genetic material into the infected cell, thus preventing viral replication. A number of side effects have been reported including neurological and gastro-intestinal complaints, Belshe, R.B., Smith, M.H., Hall, CB, Betts, Hay, R.J., Genetic basis of Resistance to Rimantidine Merging During Treatment of Influenza Virus Infection, Journ. of Virology, 1988, 62, 1508-12; Hay, A.J., The Action of Adamantanamines Against Influenza A Viruses: Inhibition of the M2 Ion Channel, Protein. Semin Virol., 1992, 3, 21-30.

Another approach to influenza treatment has been to inhibit the neuraminidase enzyme molecule on the virus, important to the virus' replication and infectivity. One such treatment drug is Zanamivir, developed by Glaxo Wellcome, Monto, A.S., Robinson, D.P., Herlocher, M.L., Hinson, J.M., Elliot, M.J., Crisp, A., Zanamivir in the prevention of influenza among healthy adults: A randomized controlled trial. JAMA, 1999, 282, 31-5. Side effects reported were sinusitis, diarrhea , nausea and adverse lung effects. A second neuraminidase inhibiting drug is Oseltamivir, licensed under the trade name Tamiflu, Treanor, J.J., Hayden, F.G., Vrooman, P.S., Bararush, R. Bettis, R., Riff, D. Efficacy and safety of the oral neuraminidase inhibitor Oseltamivir in treating acute influenza: A randomized controlled study. JAMA, 2000, 283, 1016-24. There is a concern with Amantidine, Rimantidine and Neuraminidase inhibitors that viral resistance may develop, rendering them ineffective. A. Elliot and J. Ellis, 2000, Pharmaceutical Journal, 265, 446-451.

US Patent 4,689,223 , issued August 25, 1987, assigned to T&R Chemicals, discloses compositions for treating the symptoms of or preventing the common cold nasal sprays comprising sulphites or bisulphites having low, but, not a specific pH disclosed. EP046409, published February 24, 1982, to Walliczek, discloses processes for preparation of solution of cuprous complexes for therapeutic treatment of human or animal body; incorporated herein by reference. The process discloses preparation of cuprous complex with ascorbic acid or non toxic ascorbate having a pH from 4-6. The complex may be used to make solutions for topically treating fungal, inflammatory or viral complaints. US Patent 6,080,783, issued June 27, 2000, assigned to Gum Tech International, Inc., discloses viscous gels for delivering minor effective homeopathic amount of zinc or another metal to the nasal membrane. The compositions maintain ionic zinc in direct contact with the nasal membrane and delivers rapidly zinc into the nasal membrane and into blood in those membranes for treating colds.

A known characteristic of rhinoviruses is that they lose infectivity under acidic conditions. Even pH values of 5.0 are known to reduce Rhinovirus infectivity, Hughes, J.H., Acid Lability of Rhinovirus Type 14: Effect of pH, Time and Temperature, Proc. Soc. Exp. Biol. Med., 1993, 144, 555-60. EP310317, published April 5, 1989 to Bordt et al., assigned to Beecham, discloses a method for inactivating viruses and bacteria with pharmaceutical compositions including vaccines prepared by inactivating viruses or bacteria with ascorbic acid or its salts in presence of oxygen and heavy metal ions. US Patents 4,767,788, Diana, issued August 30, 1988, assigned to Sterling Drug Inc., discloses processes for destroying viruses with glutaric acid including rhinovirus in the nasal mucosa. EP 302,772 discloses nasal administration of acid peptide compositions but does not disclose any virucidal activity.

Despite the abundance of compositions and preventative treatments known in the art, there remains a need to provide a consistent and effective method for prevention and treatment of cold and influenza symptoms.

### SUMMARY OF THE INVENTION

The present invention is for respiratory tract compositions for prevention and treatment of cold and influenza-like symptoms due to respiratory tract viral infections. The compositions of the present invention have a pH of less than 4.5, and comprise pyroglutamic acid and an organic acid having a pKa value from about 3.0 to about 5.0, wherein the combination of said pyroglutamic and organic acids provides a surface pH of the nasal cavity tissue from about pH 3.5 to 5.5. Upon application to the nasal tissues, the compositions create an environment hostile to viruses. Such an environment deters viruses from infecting the host wherein the host exhibits the symptoms mentioned above. The present invention also includes treating already infected subjects in order to mitigate the above-mentioned cold and flu symptoms. Also claimed as part of the invention are the methods of reducing or eliminating the possibility of acquisition of such viruses when confronted with a high-risk public environment including schools and office buildings.

### DEFINITIONS

The following are the definitions that should be applied to the terms used to describe the present invention:
"Respiratory tract compositions" refers to compositions in a form that is directly deliverable to the airway passages from the nose and mouth. These compositions include, but are not limited to droppers, pump sprayers, pressurized sprayers, atomizers, air inhalation devices and other packaging and equipment known or yet to be developed.
"Cold and influenza-like symptoms" refers to symptoms typically associated with respiratory tract viral infections. These symptoms include, but not limited to nasal congestion, chest congestion, sneezing, rhinorrhea, fatigue or malaise, coughing, fever, chills, body ache, sore throat and headache and other known cold and influenza-like symptoms.
" Respiratory viruses" refers to those viruses that are causal agents of cold and influenza-like symptoms. These viruses include Rhinovirus, Myxovirus (Influenza virus), Paramyxovirus (Parainfluenza virus), Respiratory Syncytial virus, Adenovirus and Coronavirus.
"Pharmaceutically acceptable vehicle" refers to any solid, liquid or gas combined with compound in the composition of the present invention to deliver the compound to the respiratory tract of the user. These vehicles are generally regarded as safe for use in humans.

### DETAILED DISCUSSION OF THE INVENTION

The compositions of the present invention comprise pyroglutamic acid and an organic acid having a pKa value from 3.0 to 5.0, wherein the combination of said pyroglutamic and organic acids provides a surface pH of the nasal cavity tissue from pH 3.5 to 5.5. The compositions have a pH of less than 4.5. Upon application to the nasal tissues, the compositions create a hostile environment to viruses. Such an environment deters one from being infected by viruses responsible for the symptoms generally recognized and mentioned above.

### Pyroglutamic Acid

The nasal compositions of the present invention comprise a safe and effective amount of pyroglutamic acid or PCA. As used herein, pyroglutamic acid collectively refers to its stereoisomers and tautomers. Pyroglutamic acid, which is also referred to as pyrrolidone carboxylic acid has two stereoisomers (D and L) and each are preferred for use herein.

The D stereoisomer of pyroglutamic acid is also known by the following names: D-Proline, 5-oxo- (+)-2-Pyrrolidone-5-carboxylic acid, (+)-Pyroglutamic acid, (R)-2-Pyrrolidone-5-carboxylic acid, 5-Oxo-D-proline, D-2-Pyrrolidone-5-carboxylic acid, D-Pyroglutamic acid, D-Pyrrolidinonecarboxylic acid, and D-Pyrrolidonecarboxylic acid.

The L stereoisomer of pyroglutamic acid is also known by the following names: L-Proline, 5-oxo- (-)-2-Pyrrolidone-5-carboxylic acid, (-)-Pyroglutamic acid, (5S)-2-Oxopyrrolidine-5-carboxylic acid, (S)-(-)-2-Pyrrolidone-5-carboxylic acid, (S)-2-Pyrrolidone-5-carboxylic acid, (S)-5-Oxo-2-pyrrolidinecarboxylic acid, (S)-Pyroglutamic acid, 2-L-Pyrrolidone-5-carboxylic acid, 2-Pyrrolidinone-5-carboxylic acid, 5-Carboxy-2-pyrrolidinone, 5-Oxo-L-proline, 5-Oxoproline, 5-Pyrrolidinone-2-carboxylic acid, Glutimic acid, Glutiminic acid, L-2-Pyrrolidone-5-carboxylic acid, L-5-Carboxy-2-pyrrolidinone, L-5-Oxo-2-pyrrolidinecarboxylic acid, L-5-Oxoproline, L-Glutamic acid, .gamma.-lactam, L-Glutimic acid, L-Glutiminic acid, L-Pyroglutamic acid, L-Pyrrolidinonecarboxylic acid, L-Pyrrolidonecarboxylic acid, Oxoproline, PCA, Pidolic acid, Pyroglutamic acid, Pyrrolidinonecarboxylic acid, Pyrrolidone-5-carboxylic acid, and Pyrrolidonecarboxylic acid.

The DL form of pyroglutamic acid (a mixture of the D and L stereoisomers) is known by the following names: DL-Proline, 5-oxo-(.+-.)-2-Pyrrolidone-5-carboxylic acid, (.+-.)-Pyroglutamic acid , 5-Oxo-DL-proline, DL-2-Pyrrolidinone-5-carboxylic acid, DL-2-Pyrrolidone-5-carboxylic acid, DL-Pyroglutamate, DL-Pyroglutamic acid, DL-Pyrrolidonecarboxylic acid, and Oxoproline. The DL form is also commercially available from Ajinomoto under the tradename Ajidew A 100 and Ajidew N 50 (Na-PCA).

Some of the above-listed stereoisomers are commercially available from UCIB, France via Bamet Products Corp., New Jersey. Such compounds are sold under trade names like Cuivridone (Cu-PCA) and L-FER Pidolate (Fe-PCA), and Pidolidone.

The compositions of the present invention comprise from 0.01% to 20% by weight of the composition of pyroglutamic acid, alternatively from 0.1% to 10%, from 0.25% to 8%, and specifically from 1% to 5%.

### Organic Acids

In addition to PCA, the present invention utilizes organic acids to create a composition hostile to the viruses mentioned above. These organic acids have a dissociation constant (pKa) from 3.0 to 5. When combined with PCA, the composition has an increased buffering capacity, providing a surface pH of the tissue treated in the nasal cavities or turbinates from 3.5 to 5.5. These organic acids are at levels from 0.01% to 10.00%, alternatively from 0.05% to 5.00% and specificaly from 0.10% to 2.50% of the composition.

Among the organic acids useful in the present invention are those selected from ascorbic acid, mono-, di-, tri- carboxylic acids and mixtures thereof. Specific mono, di or tricarboxylic acids are selected from salicylic, fumaric, benzoic, glutaric, lactic, citric, malonic, acetic, glycolic,malic, adipic, succinic, aspartic, phthalic, tartaric, glutamic, gluconic, and mixtures thereof. Use of such acids is particularly surprising to one skilled in the art in that they create this hostile environment for viruses without significantly irritating the nasal tissues the compositions they contact.

Agents are used to adjust the pH of the composition of the present invention to less than 4.5. Therefore, when the composition is applied to nasal tissues, the pH of the composition on the nasal tissues remains from 3.5 to 5.5, but is not so low as to cause irritation of the nasal tissues. Such pH- adjusting agents include those normally associated with use in topical nasal compositions including sodium bicarbonate, sodium phosphate, sodium hydroxide, ammonium hydroxide, sodium stannate, triethanolamine, sodium citrate, and combinations thereof. They may be added directly to the composition or formed by interaction within the composition during the pH adjustment process. The pH adjusting agents are generally present in an amount of from 0.01% to 5.0% by weight of the composition.

Depending on the desired form and delivery device to be used, compositions of the present invention may include pharmaceutically acceptable vehicles including co-solvents such as ethanol, propylene glycol, glycerol, water-miscible solvent; liquid aerosol propellants and mixtures thereof. Preferably these vehicles are isotonic with human plasma. Preservatives may also be included to prevent microbial contamination of dosing devices or compositions applied to the nose. Such preservatives include those normally associated with topical nasal compositions including benzalkonium chloride, chlorhexidine gluconate, phenyl ethyl alcohol, phenoxyethanol, benzyl alcohol, sorbic acid, benzoic acid, thimerosal, phenylmercuric acetate and combinations thereof.

The vehicle may also contain surfactants to facilitate virus kill as well as aid in spreading the composition throughout the respiratory tract. Gums, mucilages, thickeners, mucoadhesive polymers and mixtures thereof may be included in order to slow the normal physiologic clearance of the solution from the nasal cavity to the oropharynx. Where the composition of the present invention is in the form of a liquid, the combination of ingredients is such that the viscosity of the final composition is less than about 1000 cps at a compositional pH of about 3.5.

Volatile oils, sensates and flavors may also be included to provide desirable in-use smell and taste of the composition. Homoeopathic ingredients may also be included. A detailed, but not necessarily a complete list of such agents is found in The Homoeopathic Pharmacopoeia of the United States, 1999 ed., published by The Pharmacopoeia Convention of the American Institute of Homeopathy, ©1982, Vol. 1-4.

Suitable mucoadhesive polymers useful in the present invention exhibit pH responsiveness. By pH responsiveness, it is meant that upon contacting the mucosal fluids or tissues, the composition becomes sufficiently tacky or viscous to adhere to the tissues and not quickly erode from the surface. This is brought about by a rise in pH when the acidic product comes into contact with the less-acidic mucosal tissues and tissue fluids. This helps maintain the virus-hostile environment for longer on the mucosal surfaces than with a normal fluid product. For example, where the composition is an liquid applied using an atomizing sprayer, upon spraying into the nasal cavity, the composition quickly forms a gel like film that resists erosion due to sneezing, blowing ones nose, or mucociliary clearance.

Mucoadhesive polymers are selected from the group consisting of carboxypolymethylene; carboxyvinyl polymers; homopolymers of acrylic acid crosslinked with an allyl ether of pentaerythritol; homopolymers of acrylic acid crosslinked with an allyl ether of sucrose; homopolymers of acrylic acid crosslinked with divinyl glycol; and mixtures thereof. Homopolymers of acrylic acid crosslinked with an allyl ether of pentaerythritol or an allyl ether of sucrose are available from B. F. Goodrich Company under the tradename "Carbopol". Specific Carbopols include Carbopol 934, 940, 941, 956, 980 and mixtures thereof. An embodiment of this invention specifically utilizes Carbopol 980. Polymers of this type have slightly acidic carboxyl group substituents. Such polymers generally have a pH of around 3 in water and are generally used by neutralization during preparation of compositions to form viscous gels. Generally these polymers are used from about 0.01% to about 2.5%. Homopolymers of acrylic acid crosslinked with divinyl glycol are available from B. F. Goodrich Company as polycarbophils under the trade name "Noveon." In the present invention the mucoadhesive polymers are used in combination with the acids to form a low viscosity liquid that upon application to mucosal tissue and fluid form gels in situ.

Water may also be present in the composition of the present invention. The water, employed in the present invention should, preferably, be de-ionized and free of organic impurities. Water comprises from about 50% to 99.99%, alternatively from about 80% to about 99.95%, and specifically from about 95% to about 99.9%, by weight of the substance. This amount of water includes the free water that is added plus that amount that is introduced with other materials.

Where the composition of the present invention is a solid form the vehicle may be applied in a powder form without use of a specific vehicle. However, vehicles are often added to aid in processing of the compounds, providing acceptable flowability and a particle size of greater than 10 microns for nasal inhalant application. Other particulate or powdered pharmaceutically acceptable filler materials may be combined with compounds of the present invention to facilitate flowability, stability, handling, hygroscopicity; favorable flavor, taste and, or sensation. In the present invention, the solid vehicle is from 0% to about 99.99%, preferably from about 1% to about 99.9%, alternatively from about 10% to about 99% of the composition.

### Chelating Agents

The composition may include chelating agents to enhance antiviral activity by denying the virus metal ions. Not to be bound by theory, it is reasonable to postulate that metal cations play a major role in the formation of oxidizing species. Oxidising reactions and free radical formation can contribute to cellular damage in inflammatory diseases. Chelating agents useful in the present invention include those that chelate transition metal ions such as iron, copper, zinc and other such metals. The chelating agents useful in present invention are stable and effective in non-aqueous and aqueous medium and in pH range between 3 to 5. Specific chelating agents are selected from the group consisting of phytic acid, disodium and calcium salts of ethylene diamine tetraacetic acid (EDTA), tetrasodium EDTA, sodium hexametaphosphate (SHMP), di(hydroxyethyl)glycine,8-hydroxyquinoline and mixtures thereof. In the present invention, the chelating agents are used at levels from 0.001% to 10.00%, alternatively from 0.005% to 5.0%, and specifically from 0.01% to 2% by weight of the composition.

### Metal Salts

The nasal compositions of the present invention may comprise a safe and effective amount of a metal salt. Metal ions such as iron, silver, copper and zinc are known to exhibit antiviral properties. Zinc and its possible effects on common colds has been extensively documented, The Handbook for Curing the Common Cold, George A. Eby, published 1994, George Eby Research, Texas, USA. The mechanism of its action is thought to be multifactorial. Zinc ions have been shown to be both antiviral and antibacterial. They are believed to inhibit cleavage of rhinovirus polypeptides, preventing replication and formation of infective virions. Zinc ions reduce the ability of rhinoviruses to penetrate cell membranes, partly by lowering expression of intercellular adhesion molecule ICAM. Zinc ions have also been shown to stimulate T-cell lyphocytes, including production of the natural antiviral, interferon-gamma. They stabilize cell plasma membranes, protecting cells from cytotoxic agents, and preventing cell leakage. Metal ions may be in the form of salts or as complexes with anions.

Suitable metal salts include, but are not limited to, salts of metals selected from the groups consisting of Mn, Ag, Zn, Sn, Fe, Cu, Al, Ni, Co and combinations thereof. Preferably, the metal salts include salts of metals selected from the group consisting of Cu, Fe, Zn, and combinations thereof.

The metal salts include, but are not limited to, physiologically acceptable metal salts selected from the group consisting of salicylates, fumarates, benzoates, glutarates, lactates, citrates, malonates, acetates, glycolates, thiosalicylates, adipates, succinates, gluconates, aspartates, glycinates, tartarates, malates, maleates, ascorbates, chlorides, sulphates, nitrates, phosphates, fluorides, iodides,pidolates and combinations thereof. One embodiment is where the metal salts are selected from the group consisting of acetates, ascorbates, chlorides, benzoates, citrates, gluconates, glutarates, lactates, malates, malonates, salicylates, succinates and combinations thereof. Another alternative is wherein the metal salts are selected from the group consisting of zinc acetate, zinc chloride, zinc ascorbate, zinc gluconate, zinc pidolate and combinations thereof. Specifically the metal salt including is selected from the group consisting of acetic acid, ascorbic acid, citric acid, gluconic acid, pyroglutamic acid, glutaric acid, salicylic acid salts or metal complexes of zinc, copper, tin, silver, iron, and mixtures thereof.

Without being limited by theory, it is believed that in the compositions of the present invention, the pyroglutamic acid and metal salt complex to form a metal-acid complex that have been found to provide a synergistic immediate and residual anti-viral efficacy.

In the compositions of the present invention, the metal salt is present in amount such that the metal salt comprises from 0.001% to 20%, by weight of the composition, alternatively from 0.01% to 10%, from 0.05% to 5%, and specifically from 0.05% to 2%. Alternatively, the pyroglutamic acid and metal salt may be complexed prior to making the compositions of the present invention thereby forming a pyroglutamic acid-metal complex. In this instance, the complex is preferably present in an amount of from 0.001% to 20%, by weight of the composition, alternatively from 0.01% to 10%, and specifically from 0.1% to 5%.

### EXAMPLES

The following are non-limiting examples of compositions of the present invention. All ingredients are by weight of 100 grams of the composition:

### Example 1:

| **Component** | **% (w/w)** |
|---|---|
| PCA (Pyroglutamic Acid) | 1.00 |
| Ascorbic Acid | 1.00 |
| Phytic Acid | 1.00 |
| Mucoadhesive Polymer ¹ | 1.00 |
| Eucalyptol | 0.01 |
| Phenyl Ethyl Alcohol | 0.50 |
| Water | QS |

| | |
|---|---|
| 1. Carbopol 980 available from available from B. F. Goodrich Company | |

### Manufacturing Directions:

Disperse the Carbopol in chilled water. Add and dissolve the acidic ingredients with stirring. Premix the eucalyptol in the phenyl ethyl alcohol and add and dissolve with stirring. Adjust the pH to 3.5 with addition of sodium hydroxide.

Fill dropper vials with the solution and cap. Spray 100 microlitres of the solution into each nostril or each nostril or turbinate. Repeat three times daily.

### Example II:

| **Component** | **% (w/w)** |
|---|---|
| PCA (Pyroglutamic Acid) | 1.00 |
| Ascorbic Acid | 1.00 |
| Zinc Acetate | 0.33 |
| Mucoadhesive Polymer ¹ | 1.00 |
| Eucalyptol | 0.01 |
| Water | QS |

| | |
|---|---|
| 1. Carbopol 980 available from available from B. F. Goodrich Company | |

### Manufacturing Directions:

Disperse the Carbopol in chilled water. Add and dissolve the acidic ingredients and the zinc acetate with stirring. Premix the eucalyptol in the phenyl ethyl alcohol and add and dissolve with stirring. Adjust the pH to 3.5 with addition of sodium hydroxide.

Fill dropper vials with the solution and cap. Spray 100 microlitres of the solution into each nostril and turbinate. Repeat three times daily.

### Example III:

| **Component** | **% (w/w)** |
|---|---|
| PCA (Pyroglutamic Acid) | 1.00 |
| Citric Acid | 0.50 |
| Phytic Acid | 1.00 |
| Mucoadhesive Polymer ¹ | 1.00 |
| Eucalyptol | 0.01 |
| Phenyl Ethyl Alcohol | 0.50 |
| Water | QS |

| | |
|---|---|
| 1. Carbopol 980 available from available from B. F. Goodrich Company | |

### Manufacturing Directions:

Disperse the Carbopol in chilled water. Add and dissolve the acidic ingredients with stirring. Premix the eucalyptol in the phenyl ethyl alcohol and add and dissolve with stirring. Adjust the pH to 3.5 with addition of sodium hydroxide.

Fill dropper vials with the solution and cap. Spray 100 microlitres of the solution into each nostril or turbinate. Repeat three times daily.

### Example IV:

| **Component** | **% (w/w)** |
|---|---|
| PCA (Pyroglutamic Acid) | 1.00 |
| Citric Acid | 0.50 |
| Zinc Acetate | 0.33 |
| Mucoadhesive Polymer ¹ | 1.00 |
| Eucalyptol | 0.01 |
| Phenyl Ethyl Alcohol | 0.50 |
| Water | QS |

| | |
|---|---|
| 1. Carbopol 980 available from available from B. F. Goodrich Company | |

### Manufacturing Directions:

Disperse the Carbopol in chilled water. Add and dissolve the acidic ingredients and the zinc acetate with stirring. Premix the eucalyptol in the phenyl ethyl alcohol and add and dissolve with stirring. Adjust the pH to 3.5 with addition of sodium hydroxide.

Fill dropper vials with the solution and cap. Spray 100 microlitres of the solution into each nostril or turbinate. Repeat three times daily.

### Example V

| **Component** | **% (w/w)** |
|---|---|
| PCA (Pyroglutamic Acid) | 1.00 |
| Sodium Citrate | 0.75 |
| Eucalyptol | 0.010 |
| Ethanol | 1.00 |
| Lactose Powder | QS to 100% |

### Manufacturing Directions:

Blend the PCA and Sodium citrate together in a V-mixer to form a homogenous mix. Micronise the mixture in a fluid energy mill. Blend the micronised material by geometric addition with the lactose. Dissolve the eucalyptol in in ethanol and spray coat the powder. Evaporate the ethanol by pan drying. Blend the PCA.

Fill dry powder nasal inhalation metering pumps with the powder. Apply 10 milligrams of the powder to each nostril or turbinate.

### Example VI

| **Component** | **% (w/w)** |
|---|---|
| PCA (Pyroglutamic Acid) | 1.00 |
| Sodium Citrate | 0.75 |
| Sorbitan Trioleate | 0.40 |
| Propellants¹ | 97.85 |

| | |
|---|---|
| 1. Dupont Propellants 114 and 12 (1:1) | |

### Manufacturing Directions:

Blend the PCA and sodium citrate together in a V-mixer to form a homogenous mix. Micronise the mixture in a fluid energy mill. Dissolve the sorbitan trioleate in the mixed propellants. Disperse the PCA/Sodium citrate blend in the sorbitan trioleate/ propellant liquid. Fill the suspension into pressurized metered dose inhalers using standard filling techniques. Administer 100 microlitres from the metered dose inhaler into each nostril or turbinate.

## Claims

1. A low irritation nasal composition for prevention and treatment of cold and influenza viruses comprising pyroglutamic acid and an organic acid having a dissociation constant (pKa) value from 3.0 to 5.0, wherein the composition has pH of less than 4.5 and the combination of said pyroglutamic and organic acids provides a surface pH of the nasal cavity tissue from pH 3.5 to 5.5.

2. The composition according to Claim 1 comprising 0.01% to 20% of pyroglutamic acid.

3. The composition according to Claim 1 or Claim 2 wherein the organic acid is selected from ascorbic acid, mono-, di-, tri- carboxylic acids and mixtures thereof, preferably selected from salicylic, fumaric, benzoic, glutaric, lactic, citric, malonic, acetic, glycolic, malic, adipic, succinic, aspartic, phthalic, tartaric, glutamic, gluconic, and mixtures thereof.

4. The composition according to any preceding claim which comprises from 0.01% to 10% of the organic acid.

5. The composition according to any preceding claim comprising from 0.01% to 10% of a chelating agent, preferably wherein the chelating agent is selected from phytic acid, disodium and calcium salts of ethylene diamine tetraacetic acid (EDTA), tetrasodium EDTA, sodium hexametaphosphate (SHMP), di(hydroxyethyl)glycine, 8-hydroxyquinoline and mixtures thereof.

6. The composition according to any preceding claim comprising metal salts, preferably from 0.01% to 10% metal salts, more preferably metal salts selected from acetates, ascorbates, chlorides, benzoates, citrates, gluconates, glutarates, lactates, malates, malonates, salicylates, succinates and combinations thereof.

7. The composition according to any preceding claim comprising a mucoadhesive agent, preferably wherein the mucoadhesive agent is selected from carboxypolymethylene; carboxyvinyl polymers; homopolymers of acrylic acid crosslinked with an allyl ether of pentaerythritol an allyl ether of sucrose or divinyl glycol; and mixtures thereof.

8. The use of pyroglutamic acid in the manufacture of a composition according. to any preceding claim for preventing or treating cold and influenza viruses by spraying the composition into the nasal turbinates.

## Patentansprüche

1. Nasalzusammensetzung mit geringfügiger Reizung zur Vorbeugung und Behandlung von Erkältung und Influenzaviren, umfassend Pyroglutaminsäure und eine organische Säure mit einem Dissoziationkonstantenwert (pKa) von 3,0 bis 5,0, worin die Zusammensetzung einen pH von weniger als 4,5 aufweist und die Kombination der Pyroglutaminsäure und der organischen Säure einen Oberflächen-pH des Nasenhöhlengewebes von pH 3,5 bis 5,5 zur Verfügung stellt.

2. Zusammensetzung nach Anspruch 1, umfassend 0,01% bis 20% Pyroglutaminsäure.

3. Zusammensetzung nach Anspruch 1 oder Anspruch 2, worin die organische Säure ausgewählt ist aus Ascorbinsäure, Mono-, Di-, Tricarbonsäuren und Mischungen davon, vorzugsweise ausgewählt aus Salicylsäure, Fumarsäure, Benzoesäure, Glutarsäure, Milchsäure, Zitronensäure, Malonsäure, Essigsäure, Glykolsäure, Äpfelsäure, Adipinsäure, Bernsteinsäure, Asparaginsäure, Phthalsäure, Weinsäure, Glutaminsäure, Gluconsäure und Mischungen davon.

4. Zusammensetzung nach einem der vorangehenden Ansprüche, welche 0,01% bis 10% organische Säure umfasst.

5. Zusammensetzung nach einem der vorangehenden Ansprüche, umfassend 0,01% bis 10% eines Komplexierungsmittels, vorzugsweise worin das Komplexierungsmittel ausgewählt ist aus Phytinsäure, Dinatrium- und Calciumsalzen von Ethylendiamintetraessigsäure (EDTA), Tetranatrium-EDTA, Natriumhexametaphosphat (SHMP), Di(hydroxyethyl)glycin, 8-Hydroxychinolin und Mischungen davon.

6. Zusammensetzung nach einem der vorangehenden Ansprüche, umfassend Metallsalze, vorzugsweise 0,01% bis 10% Metallsalze, besonders bevorzugt Metallsalze, ausgewählt aus Acetaten, Ascorbaten, Chloriden, Benzoaten, Citraten, Gluconaten, Glutaraten, Lactaten, Malaten, Malonaten, Salicylaten, Succinaten und Kombinationen davon.

7. Zusammensetzung nach einem der vorangehenden Ansprüche, umfassend ein mucoadhäsives Mittel, vorzugsweise worin das mucoadhäsive Mittel ausgewählt ist aus Carboxypolymethylen; Carboxyvinylpolymeren; Homopolymeren von Acrylsäure, vernetzt mit einem Allylether von Pentaerythrit, einem Allylether von Saccharose oder Divinylgykol; und Mischungen davon.

8. Verwendung von Pyroglutaminsäure in der Herstellung einer Zusammensetzung gemäß einem der vorangehenden Ansprüche zur Vorbeugung oder Behandlung von Erkältung und Influenzaviren durch Sprühen der Zusammensetzung in die Nasenmuschel.

## Revendications

1. Composition nasale faiblement irritante pour la prévention et le traitement des virus du rhume et de la grippe, comprenant de l'acide pyroglutamique et un acide organique ayant une valeur de constante de dissociation (pKa) de 3,0 à 5,0, la composition ayant un pH inférieur à 4,5 et la combinaison desdits acides pyroglutamique et organique fournit un pH superficiel des tissus des fosses nasales de 3,5 à 5,5.

2. Composition selon la revendication 1, comprenant 0,01 % à 20 % d'acide pyroglutamique.

3. Composition selon la revendication 1 ou 2, dans laquelle l'acide organique est choisi parmi l'acide ascorbique, les acides mono-, di-, tri- carboxyliques et leurs mélanges, de préférence parmi les acides salicylique, fumarique, benzoïque, glutarique, lactique, citrique, malonique, acétique, glycolique, malique, adipique, succinique, aspartique, phtalique, tartrique, glutamique, gluconique, et leurs mélanges.

4. Composition selon l'une quelconque des revendications précédentes, qui comprend 0,01 % à 10 % de l'acide organique.

5. Composition selon l'une quelconque des revendications précédentes, comprenant 0,01 % à 10 % d'un agent chélatant, l'agent chélatant étant, de préférence, choisi parmi l'acide phytique, les sels disodique et calcique de l'acide éthylènediaminetétraacétique (EDTA), l'EDTA tétrasodique, l'hexamétaphosphate de sodium (SHMP), la di(hydroxyéthyl)glycine, la 8-hydroxyquinoléine, et leurs mélanges.

6. Composition selon l'une quelconque des revendications précédentes, comprenant des sels métalliques, de préférence 0,01 % à 10 % de sels métalliques, mieux encore des sels métalliques choisis parmi les acétates, les ascorbates, les chlorures, les benzoates, les citrates, les gluconates, les glutarates, les lactates, les malates, les malonates, les salicylates, les succinates, et leurs combinaisons.

7. Composition selon l'une quelconque des revendications précédentes, comprenant un agent mucoadhésif, l'agent mucoadhésif étant, de préférence, choisi parmi le carboxypolyméthylène ; les polymères carboxyvinyliques ; les homopolymères d'acide acrylique réticulés avec un éther allylique de pentaérythritol, un éther allylique de saccharose ou de divinylglycol ; et leurs mélanges.

8. Utilisation d'acide pyroglutamique dans la fabrication d'une composition selon l'une quelconque des revendications précédentes pour la prévention ou le traitement de virus du rhume et de la grippe en pulvérisant la composition dans les fosses nasales.
